# EUROPÄISCHE PATENTANMELDUNG

(11) **EP 0 677 280 A1**
(43) Veröffentlichungstag der Anmeldung: **18.10.1995**
(21) Anmeldenummer: 94118352.7
(22) Anmeldetag: 22.11.1994
(51) Int. Cl.: A61F 2/34, A61F 2/30

(54) **Hüftpfannen-Prothese**

(30) Priorität: 15.04.1994 DE 4413038
(71) Anmelder: ALPHANORM MEDIZINTECHNIK GmbH, D-66538 Neunkirchen (DE)
(72) Erfinder: Krauspe, Rüdiger, Dr., D-97078 Würzburg (DE)
(74) Vertreter: Hansmann, Axel, Dipl.-Wirtsch.-Ing.

(57) **Zusammenfassung**

Die Erfindung betrifft eine Hüftpfannen-Prothese (1) mit einem im wesentlichen sphärischen, festen Prothesenkörper (3) zum Verwachsen mit dem Knochenbau des Prothesenträgers und mit Durchgangslöchern (13,15) für in den Knochenbau eintreibbaren Befestigungselementen (23), wobei die Innenfläche des Prothesenkörpers ein Inlay aufweist, an welchem der Femur-Kopf gleiten kann. Es ist vorgesehen, daß ein oder mehrere Durchgangslöcher (13,15) des Prothesenkörpers (3) für die Aufnahme von Verschlußstopfen (29,31) ausgestaltet sind, welche gegen den Durchtritt von Blut zum Inlay abdichten und von der Innenfläche des Prothesenkörpers (3) her herausnehmbar sind. Dabei sind die Verschlußstopfen (29,31) als Schraubstopfen ausgestaltet, deren Kopf mit einer Inbus-Senkung (39,41) für ein Schraubwerkzeug ausgerüstet ist.

## Beschreibung

Die Erfindung betrifft eine Hüftpfannen-Prothese gemäß dem Oberbegriff des Anspruches 1.

Die DE-PS 21 38 881 beschreibt eine Hüftpfannen-Prothese der eingangs als bekannt vorausgesetzten Art, die zum Einwchsen in den Knochenbau des Prothesenträgers vorgesehen ist, wobei aber zur Lagesicherung der Prothese auch Befestigungselemente durch Löcher des festen Prothesenkörpers in den Beckenknochen eintreibbar sind.

Zur Lagensicherung einer Hüftpfannen-Prothese an dem Beckenknochen des Prothesenträgers sind bereits verschiedene Vorkehrungen bekannt geworden. So zeigt die DE-OS 35 18 246 außen an der Hüftpfanne angebrachte Befestigungsrippen, und bei der DE-OS 33 22 978 ist die dem Beckengewebe zugekehrte Außenseite der Hüftpfanne mit über ihre äußere Umfangsfläche verteilt angeordneten Ausnehmungen versehen, in denen jeweils ein Pfropfen aus einem Material angeordnet ist, welches im implantierten Zustand wenigstens teilweise löslich ist. Bei dieser bekannten Hüftpfannen-Prothese werden die teilweise löslichen Pfropfen in Sacklöchern vorgesehen, welche einen Durchmesser von beispielsweise 2 - 4 mm sowie eine Tiefe von 3 mm aufweisen, aber nicht bis zur Innenseite der Hüftpfannen-Prothese durchgehen. Dabei ist auch vorgesehen, daß die Ausnehmungen an ihrer Innenseite mit einer Profilierung versehen sind, um die einzusetzenden Pfropfen durch Reibschluß und einen gewissen Formschluß in den Ausnehmungen zu halten. Die Profilierung soll dabei beispielsweise in der Form eines Gewindes, konzentrischer Ringe oder Rillen ausgestaltet sein. Die eingesetzten Pfropfen sind dabei aus einem Material, welches sich im implantierten Zustand unter Einwirkung des natürlichen Gewebes im Verlaufe der Zeit wenigstens zum Teil gelöst und so dem im Verlaufe der Zeit entstehenden neuen Gewebe die Möglichkeit gibt, in die Ausnehmungen in entsprechendem Ausmaße hineinzuwachsen.

Derartige Hüftpfannen-Prothesesn sollen einerseits ohne die Verwendung von Zement anbringbar sein, weil Zement möglicherweise zu einem früheren Zeitpunkt eine Re-Operation notwendig macht, und es sollen zum anderen auch gute Stabilität der Hüftpfanne durch Verwendung eines starren Prothesenkörpers und günstige Gleiteigenschaften der Gelenkkugel des Femur-Kopfes an der inneren Oberfläche der Hüftpfannen-Prothese gegeben sein. Diesem Zwecke dient der bekannte schichtweise Aufbau von Hüftpfannen-Prothesen.

Die Eintreibung von Befestigungselementen, wie Nägeln oder Schrauben durch den festen Prothesenkörper in den Beckenknochen ist zur Lagesicherung der Prothese in vielen Fällen unvermeidbar, und es müssen daher an den festen Prothesenkörper praktisch bereits Aufnahmelöcher für solche Befestigungselemente vorgesehen sein. Dabei ist es auch erforderlich, mehrere solche Aufnahmelöcher vorzusehen, um ein oder mehrere Befestigungselemente je nach der angetroffenen Situation an dieser ohne jener Stelle vorsehen zu können.

Der vorliegenden Erfindung liegt die Aufgabe zugrunde, bei Hüftpfannen-Prothesen der in Rede stehenden Art dem Umstand Rechnung zu tragen, daß vor einer Operation zur Implantation einer solchen Prothese im allgemeinen nicht absehbar ist, an welchen Stellen denn eine zusätzliche Sicherung der Prothese durch in den Knochen einzutreibende Befestigungselemente notwendig ist. Es soll insbesondere auch vermieden werden, daß ein an der Hüftpfannen-Prothese zur Verbesserung der Gleiteigenschaften mit der Gelenkkugel des Femur-Kopfes vorgesehenes Inlay übermäßigen Kontakt mit dem Blut bekommt.

Die Lösung der Aufgabe ist ausgehend von einer Hüftpfannen-Prothese der eingangs als bekannt vorausgesetzten Gattung in dem Patentanspruch 1 gekennzeichnet.

Eine solche Hüftpfannen-Prothese zeichnet sich dadurch aus, daß sie primär als komplett verschlossene Schale implantiert werden kann. Erübrigt sich eine zusätzliche Sicherung mittels Befestigungsschrauben, bleibt die geschlossene Schalenform erhalten und wird Kontakt eines eingesetzten Kunststoff-Inlays mit dem Blut ohne weiteres vermieden. Wird doch eine zusätzliche Schraubenfixation notwendig, dann dreht man an den gewünschten Stellen einfach einen oder mehrere Stopfen heraus, um die Schrauben in der erforderlichen Weise anzubringen.

Gemäß Anspruch 2 werden die Verschlußstopfen selbst als Schraubstopfen ausgestaltet, wobei die Ansprüche 3 und 4 alternative Anbringungsmöglichkeiten für das Gewinde der Schraubstopfen kennzeichnen.

Anspruch 5 kennzeichnet die Anordnung einer eingesenkten Angriffsfläche für ein Schraubwerkzeug, wobei eine Inbus-Senkung den Vorteil der Aufrechterhaltung möglichst guter Dichtung zwischen den Umfangsflächen des Pfropfens und den zugehörigen Anlageflächen des Durchgangsloches des Prothesen-Körpers hat.

Es folgt die Beschreibung eines Ausführungsbeispieles der Erfindung anhand von Zeichnungen.

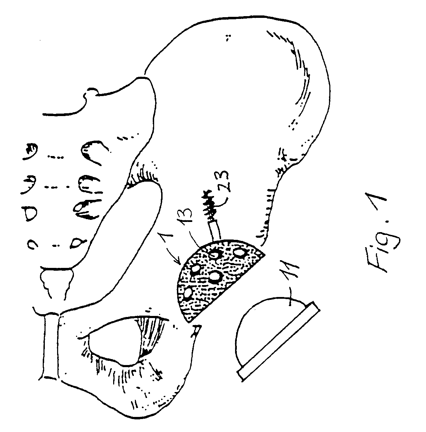

- Fig. 1: zeigt schematisch einen menschlichen Beckenknochen mit eingesetzter Hüftpfpannenprothese mit Andeutung verschiedener Durchgangslöcher und einer in den Beckenknochen eingetriebenen Befestigungsschraube, sowie auch (in Abstand) einem in die Hüftpfannen-Prothese einzusetzenden Kunststoff-Inlay mit günstigen Reibungseigenschaften gegenüber der Gelenkkugel des Oberschenkel-Knochens (Femur-Kopf).
- Fig. 2: zeigt den Prothesenkörper der Hüftpfannen-Prothese schematisch im Schnitt einschließlich mit Gewinde versehenen Durchgangslöchern für möglicherweise anzubringende Befestigungsschrauben bzw. Verschlußstopfen.
- Fig. 3: zeigt eine erste Ausführungsform eines Verschlußstopfens, der in ein Schrauben-Aufnahmeloch des Prothesenkörpers einsetzbar ist, wenn eine Schraube nicht notwendig ist.
- Fig. 4: zeigt eine andere Ausführungsform eines solchen Verschlußstopfens.
- Fig. 5: zeigt einen Grundriß des Prothesenkörpers aus Fig. 2.

Die in den Zeichnungen gezeigte Hüftpfannen-Prothese 1 hat einen im wesentlichen sphärischen Prothesenkörper 3 aus starkwandigem Metall. Die äußere Oberfläche 5 des Prothesenkörpers weist geeignete Strukturierung zum Verwachsen des Prothesenkörpers mit dem Knochen auf. Das bedarf hier keiner näheren Beschreibung, und die Einbaulage des Prothesenkörpers erfolgt gemäß den dargestellten und bekannten anatomischen Gegebenheiten.

Die sphärische Innenfläche 7 des Prothesenkörpers weist am Umfang eine Stufung 9 auf, die zur Lagensicherung eines Kunststoff-Inlays 11 dient. Ein solches Inlay dient der Verbesserung der Lagerung der nicht näher dargestellten Gelenkkugel des Oberschenkel-Knochens. Es ist erwünscht, einen übermäßigen Kontakt des Kunststoff-Inlays mit Blut zu vermeiden.

An dem Prothesenkörper sind mehrere Durchgangslöcher der bei 13 und 15 gezeigten Art vorgesehen. Das bei 13 gezeigte Durchgangsloch ist eines einer größeren Anzahl von Durchgangslöchern, die beispielsweise auf zwei konzentrischen Reihen wie durch die Kreuze 17 und 19 angedeutet, angeordnet sind. Diese Durchgangslöcher können möglicherweise als Schraubenaufnahme-Löcher dienen, wenn sich bei der Operation herausstellt, daß die Hüftpfannen-Prothese nicht nur durch Zusammenwachsen mit dem Knochen, sondern zusätzlich durch ein oder mehrere Schrauben wie bei 23 gezeigt, gesichert werden soll.

Das Durchgangsloch 15 dient zur Befestigung eines nicht näher gezeigten Einschlagstabes, der zeitweilig mit dem Gewinde 25 des Durchgangsloches 15 verschraubt werden kann, so daß der Operateur die Hüftpfannen-Prothese zunächst mittels des Einschlagstabes handhaben und in die in den Knochen vorbereitete Öffnung einschlagen und darin ausrichten kann.

Auch die Schraubenaufnahme-Löcher 13 sind mit Gewinde 27 ausgerüstet.

Die Gewinde 25 und 27 der Durchgangslöcher 13 und 15 dienen zur Befestigung von Verschlußstopfen 29 und 31 der in den Figuren 3 und 4 gezeigten Art. Diese haben den Gewinden 25 und 27 der Durchgangslöcher 15 und 13 entsprechende Gewindeteile 33 und 35. Das Gewinde 33 sitzt an dem Schaftteil des Verschlußstopfens 31. Das Gewinde 25 des Durchgangsloches 15 befindet sich innerhalb dessen eigentlicher Durchtrittsöffnung, die kleineren Durchmesser als eine kegelige Einsenkung 35 hat, die dem kegeligen Kopf 37 des als Schraubstopfen ausgestalteten Verschlußstopfens 31 (Fig. 4) dient.Die kegelige Gestaltung kann derjenigen des hier nicht näher gezeigten Einschlagstabes entsprechen.

Das Gewinde 27 des Durchgangsloches 13 befindet sich am Außenumfang einer vergrößerten Durchmesser aufweisenden zylindrischen Einsenkung dieses Durchgangsloches, die dem zylindrisch ausgestalteten Kopf des Schraub-Verschlußstopfens 29 entspricht.

Im Anlieferzustand können sämtliche Durchgangslöcher 13, 15, 17, 19 des Prothesenkörpers mittels der Verschlußstopfen 27 und 31 verschlossen sein, so daß die Hüftpfanne primär als komplett verschlossene Schale implantiert werden kann. Die Innenseite kann auf diese Weise von Blut freigehalten und ein einzusetzendes Inlay der bei 11 gezeigten Art vor Blut geschützt bleiben.

Erweist es sich dann als notwendig, eine Befestigungsschraube 13 oder auch einen Einschlagstab anzubringen, so kann an der betreffenden Stelle der dort befindliche Verschlußstopfen herausgeschraubt werden. Das so entstehende Loch wird im übrigen dann durch die Befestigungsschraube auch wieder verschlossen.

Als Angriffsfläche für ein Einschraubwerkzeug der Verschlußstopfen bewährt sich besonders eine Inbus-Senkung, wie bei 39 bzw. 41 gezeigt. Eine solche Inbus-Senkung verletzt den Außenumfang des Kopfes des Verschlußstopfens nicht und gewährleistet somit günstige Abdichtung.

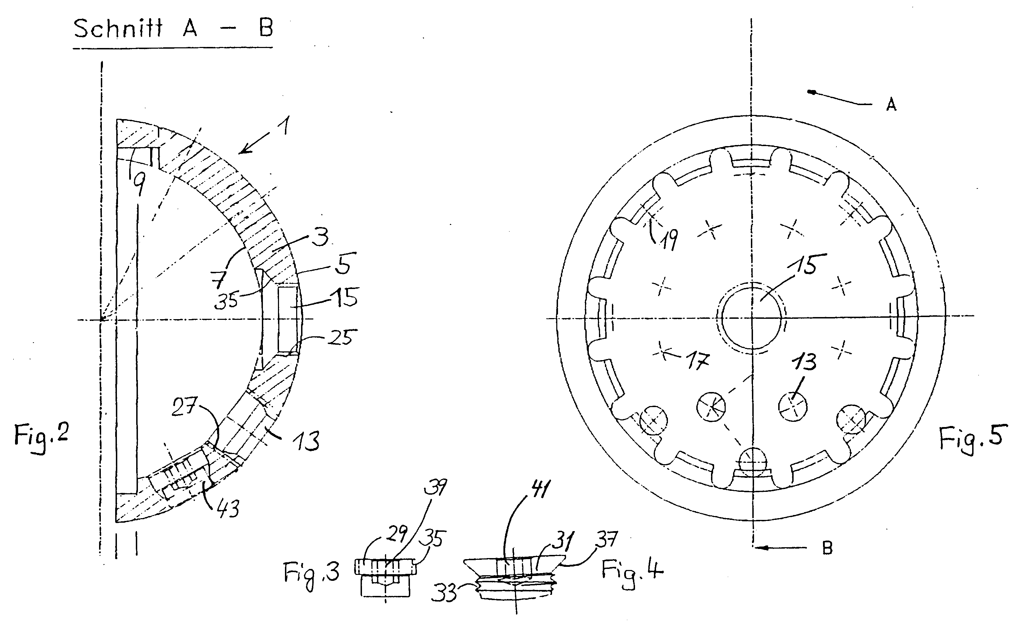

In Fig. 2 sind die Durchgangslöcher 13 und 15 jeweils offen gezeigt, und zum Verschließen eignen sich die in den Figuren 3 und 4 gezeigten Schraub-Verschlußstopfen 29 und 31.

Am unteren Ende der Figur 2 sieht man einen in ein Durchgangsloch bereits eingesetzten Schraub-Verschlußstopfen 43.

Abmessung und Länge der Verschlußstopfen können so gewählt sein, daß sie im wesentlichen mit der Innenfläche und der äußeren Oberfläche des Prothesenkörpers bündig sind.

## Patentansprüche

1. Hüftpfannen-Prothese mit einem im wesentlichen sphärischen, festen Prothesenkörper zum Verwachsen mit dem Knochenbau des Prothesenträgers und mit Durchgangslöchern für in den Knochenbau eintreibbaren Befestigungselementen, wobei die Innenfläche des Prothesenkörpers ein Inlay aufweist, an welchem der Femur-Kopf gleiten kann,
**dadurch gekennzeichnet,** daß ein oder mehrere Durchgangslöcher (13, 15) des Prothesenkörpers für die Aufnahme von Verschlußstopfen (29,31) ausgestaltet sind, welche gegen den Durchtritt von Blut zum Inlay (11) abdichten und von der Innenfläche (7) des Prothesenkörpers her herausnehmbar sind.

2. Hüftpfannen-Prothese nach Anspruch 1,
**dadurch gekennzeichnet,** daß die Verschlußstopfen als Schraubstopfen (29, 31) ausgestaltet sind.

3. Hüftpfannen-Prothese nach Anpsruch 1 oder 2,
**dadurch gekennzeichnet,** daß an einem Durchgangsloch (13) eine zylindrische Senkung für einen zylindrischen Kopfteil eines Verschlußstopfens (29) vorgesehen ist, und ein mit dem Gewinde (35) des Verschlußstopfens zusammenpassendes Gewinde (27) aufweist.

4. Hüftpfannen-Prothese nach Anpsruch 1 oder 2,
**dadurch gekennzeichnet,** daß die Durchtrittsöffnung eines Durchgangsloches (15) des Prothesenkörpers und ein schaftteil des Verschlußstopfens mit zusammenpassenden Gewindegängen (25,33) ausgestattet sind.

5. Hüftpfannen-Prothese nach Anspruch 3 oder 4,
**dadurch gekennzeichnet,** daß der Kopf des Verschlußstopfens (29 bzw. 31) mit einer Inbus-Senkung (39, 41) für ein Schraubwerkzeug ausgestattet ist.

6. Hüftpfannen-Prothese nach einem oder mehreren der voranstehenden Ansprüche, **dadurch gekennzeichnet,** daß mehrere Durchgangslöcher (13), die zur Aufnahme von in den Knochen einzutreibenden Schrauben (23) ausgestaltet sind versetzt auf zwei etwa konzentrischen Kreisen (17, 19) um den Umfang der Prothese verteilt sind.

7. Hüftpfannen-Prothese nach einem oder mehreren der voranstehenden Anpsrüche, **dadurch gekennzeichnet,** daß ein Durchgangsloch (15) für die Aufnahme eines beim Anbringen der Prothese am Knochen zu verwendenden Einschlagstabes ausgestaltet ist.
